(19) Europäisches Patentamt
European Patent Office
Office européen des brevets

(11) Veröffentlichungsnummer : **0 431 265 B1**

(12) **EUROPÄISCHE PATENTSCHRIFT**

(45) Veröffentlichungstag der Patentschrift :
**03.11.93 Patentblatt 93/44**

(51) Int. Cl.$^5$ : **C07C 15/14, C07C 2/86**

(21) Anmeldenummer : **90117140.5**

(22) Anmeldetag : **06.09.90**

(54) **Verfahren zur Herstellung von p-Aralkylbiphenylen.**

(30) Priorität : **06.12.89 DE 3940331**

(43) Veröffentlichungstag der Anmeldung :
**12.06.91 Patentblatt 91/24**

(45) Bekanntmachung des Hinweises auf die
Patenterteilung :
**03.11.93 Patentblatt 93/44**

(84) Benannte Vertragsstaaten :
**AT BE CH DE DK ES FR GB GR IT LI LU NL SE**

(56) Entgegenhaltungen :
**FR-A- 2 298 526**

(73) Patentinhaber : **RÜTGERSWERKE
AKTIENGESELLSCHAFT
Mainzer Landstrasse 217
D-60326 Frankfurt (DE)**

(72) Erfinder : **Haase, Jürgen, Dr.
Mozartstrasse 19
D-4355 Waltrop (DE)**
Erfinder : **Hillner, Knut, Dr.
Baroperstrasse 354
D-4600 Dortmund (DE)**
Erfinder : **Brüggemann, Wolfgang
Gemeindeplatz 3
D-4620 Castrop-Rauxel (DE)**
Erfinder : **Momm, Gabriele
Suderwicher Strasse 143
D-4350 Recklinghausen (DE)**

EP 0 431 265 B1

## Beschreibung

Die Erfindung betrifft ein verbessertes Verfahren zur Herstellung von p-Aralkylbiphenylen insbesondere von p-Benzylbiphenyl. Reine p-Aralkylbiphenyle finden Verwendung als Sensibilisierungsmittel in Thermokopiersystemen, während verunreinigte Aralkylbiphenyle, insbesondere solche, die größere Mengen an o- oder m-Isomeren enthalten, für diese Anwendung nicht geeignet sind. Zudem wird die Aufarbeitung des Isomerengemisches zwecks Gewinnung des reinen p-Isomeren umso schwieriger, je größer der Anteil an o- und m-Isomeren im Isomerengemisch ist.

Aus JP-A 74 104 890 (C.A. 82:125044s) ist bekannt, p-Benzylbiphenyl durch Umsetzung von Biphenyl mit Benzylchlorid in Anwesenheit von Friedel-Crafts-Katalysatoren herzustellen. Erhalten wird z. B. bei einer Umsetzung mit $AlCl_3$ als Katalysator ein Isomerengemisch. Eigene Versuche zeigten, daß im Isomerengemisch die o- und p-Isomeren im molaren Verhältnis 1 : 0,8 vorliegen.

Es ist daher die Aufgabe der vorliegenden Erfindung, ein wirtschaftliches Verfahren zur Herstellung von Aralkylbiphenylen bereitzustellen, bei dem der Anteil an entstehendem p-Isomeren im Vergleich zu den m- und o-Isomeren möglichst hoch ist.

Die Lösung der Aufgabe erfolgt durch ein Verfahren gemäß der Ansprüche 1 bis 5.

Nach EP-A 0 285 280 wird Biphenyl mit olefinisch ungesättigten Verbindungen alkyliert, wobei als Katalysator ein Zeolith dient mit einem molaren Verhältnis von $SiO_2 : Al_2O_3$ von nicht weniger als 10. Bei dieser Umsetzung entstehen p-Alkylderivate in hoher Selektivität.

Die Übertragung dieses Verfahrens auf die Aralkylierung von Biphenyl ist jedoch nicht vorteilhaft. Es wird ein Isomerengemisch erhalten, in dem mehr m- und o-Isomere enthalten sind als p-Isomer.

Überraschenderweise wurde jedoch gefunden, daß sich das Verhältnis der bei der Aralkylierung von Biphenyl erhaltenen Aralkylbiphenyle stark zugunsten der p-Isomeren verschiebt, wenn die Reaktion an einem Katalysator vom Montmorillonit-Typ erfolgt, der eine spezifische Oberfläche von mindestens 110 $m^2$/g hat. Die spezifische Oberfläche der erfindungsgemäß einzusetzenden Katalysatoren liegt im allgemeinen im Bereich von 150 bis 280 $m^2$/g, insbesondere im Bereich von 180 bis 220 $m^2$/g oder im Bereich von 220 bis 270 $m^2$/g.

Die Durchführung des Verfahrens erfolgt in an sich bekannter Weise durch Umsetzung der Reaktanten miteinander in Gegenwart des Katalysators und anschließender Aufarbeitung des Reaktionsgemisches.

Reaktanten sind Biphenyl einerseits und Aralkylierungsmittel andererseits. Aralkylierungsmittel sind aromatische Verbindungen, die außer dem aromatischem Kern eine Alkylgruppe mit einer reaktiven Gruppe haben. Der aromatische Kern, Benzyl- oder Naphthylkern, kann unsubsitutiert oder mit einer oder mehreren Alkyl-, Halogenid-, Hydroxy-, Methoxy- oder Aminogruppen substituiert sein. Er muß zusätzlich eine Alkylgruppe mit einer reaktiven Gruppe enthalten.

Entsprechende reaktive Gruppen sind Halogenide, Hydroxy- oder olefinisch ungesättigte Gruppen.

Bevorzugte Beispiele für erfindungsgemäß einzusetzende Aralkylierungsmittel sind Benzylhalogenide, insbesondere Benzylchlorid, Benzylalkohol, 1-Phenylethanol, Naphthylmethanol, Styrol oder alpha-Methylstyrol.

Biphenyl und Aralkylierungsmittel können in äquimolaren Mengen eingesetzt werden. Bevorzugt wird jedoch ein mehrfacher molarer Überschuß von Biphenyl eingesetzt, da dadurch unerwünschte Nebenreaktionen wie z. B. Mehrfacharalkylierungen reduziert werden. Die bevorzugt eingesetzten molaren Mengen von Biphenyl und Aralkylierungsmitteln liegen im Bereich von 2 : 1 bis 4 : 1.

Die Reaktanten gelangen entweder flüssig (aufgeschmolzen) oder in Lösung zum Einsatz. Als entsprechende Lösemittel eignen sich solche, die gegenüber den Reaktanten inert sind und die ein gutes Lösevermögen haben. Beispiele hierfür sind Acetonitril, Nitrobenzol, Dimethylsulfoxid, Cyclohexan oder Benzin.

Die erfindungsgemäße Reaktion kann sowohl kontinuierlich als auch diskontinuierlich durchgeführt werden.

Im einfachsten Fall werden Reaktanten und Katalysatoren in einem Reaktor unter Rühren miteinander vermischt und während einer Reaktionszeit von 1 bis 10 h unter Rühren auf einer Reaktionstemperatur im Bereich von 70 bis 240 °C gehalten. Die Menge des eingesetzten Katalysators liegt im Bereich von 2,5 bis 50 Gew.-%, bevorzugt von 5 bis 20 %, bezogen auf eingesetztes Biphenyl.

Da der Katalysator nach der Umsetzung wieder gereinigt und neu verwendet werden kann, sind die hohen Katalysatormengen nicht störend.

Die Reaktionstemperatur muß bei lösemittelfreier Umsetzung über dem Schmelzpunkt des Reaktanten mit dem höchsten Schmelzpunkt - meist dem Schmelzpunkt des Biphenyls (69 °C) liegen. Eine Erhöhung der Reaktionstemperatur verkürzt die Reaktionszeit, erhöht aber auch die unerwünschten Nebenreaktionen. Im allgemeinen wird man bei alkoholischen Aralkylierungsmitteln aus Gründen der geringeren Reaktivität eine höhere Reaktionstemperatur wählen, bevorzugt im Bereich von 150 bis 220 °C, als bei olefinisch ungesättigten oder alkylhalogenidhaltigen Aralkylierungsmitteln bei denen eine Umsetzung im Bereich von 80 bis 140 °C be-

2

vorzugt wird.

Die verwendeten Katalysatoren sind im Handel erhältliche Ton-Katalysatoren vom Montmorillonit-Typ.

Es wurde gefunden, daß die Selektivität bezüglich des p-Isomeren mit zunehmender spezifischer Oberfläche der Montmorillonit-Katalysatoren zunimmt. Ungeeignet sind solche mit einer spezifischen Oberfläche von weniger als 110 m²/g. Gute Ergebnisse werden erhalten, wenn die spezifische Oberflächen im Bereich von 110 bis 300 m²/g bervorzugt im Bereich von 180 bis 280, insbesondere im Bereich von 220 bis 270 m²/g liegt.

Die Katalysatoren können pulverförmig oder in granulierter Form eingesetzt werden. Nach erfolgter Reaktion werden die Katalysatoren in an sich bekannter Weise wie etwa durch Zentrifugieren oder Filtrieren vom Reaktionsgemisch abgetrennt, mit polaren Lösemitteln gewaschen und getrocknet. Sie können danach erneut zur Aralkylierung von Biphenyl eingesetzt werden.

**Beispiele**

Beispiel 1

In einem heizbaren Rührreaktor werden 154 g (1 mol) geschmolzenes Biphenyl und 63 g (0,5 mol ) Benzylchlorid mit 31 g (etwa 20 Gew.-%, bezogen auf Biphenyl ) eines Montmorillonitpulvers mit einer spezifischen Oberfläche von 220 bis 270 m²/g vermischt und bei 110 °C 2 h lang gerührt. Danach wird der Katalysator abfiltriert und das entstande Reaktionsgemisch analysiert.

Die erhaltenen Ergebnisse sind in Tabelle I niedergelegt.

Beispiele 2 - 17

Analog zu Beispiel 1 wird die Benzylierung von Biphenyl durchgeführt, wobei jeweils 1 Parameter verändert wird. Die jeweiligen Änderungen und die erhaltenen Ergebnisse sind in Tabelle I niedergelegt.

Beispiele 18 und 19 (Vergleichsbeispiele)

Analog zu Beispiel 1 wird die Benzylierung von Biphenyl durchgeführt, wobei nicht erfindungsgemäße Katalysatoren eingesetzt werden.

In Beispiel 18 wird ein Zeolith ZSM-5 eingesetzt.

In Beispiel 19 wird ein Montmorillonit mit einer spezifischen Oberfläche von weniger als 110 eingesetzt.

Die erhaltenen Ergebnisse sind in Tabelle I niedergelegt.

EP 0 431 265 B1

Tabelle I

| Bei-spiel | Reaktions-temperatur [°C] | dauer [h] | Mol-Verhältnis Biphenyl/ Benzylchlorid | Katalysator, spezifische Oberfläche [m²/g] | Umsatz Benzyl-chlorid [mol-%] | Biphenyl [mol-%] | Ausbeute an Benzylbiphenyl p- [mol-%] bez. a. Benzylchlorid | m- | o- |
|---|---|---|---|---|---|---|---|---|---|
| 1 | 110 | 2 | 2 : 1 | 220 - 270 | 100 | 54,4 | 25,9 | 6,9 | 1,0 |
| 2 | 120 | 2 | 2 : 1 | 220 - 270 | 100 | 50,5 | 30,8 | 7,5 | 1,3 |
| 3 | 130 | 2 | 2 : 1 | 220 - 270 | 100 | 50,0 | 35,7 | 6,6 | 2,3 |
| 4 | 140 | 2 | 2 : 1 | 220 - 270 | 100 | 51,6 | 24,3 | 8,9 | 0,9 |
| 5 | 150 | 2 | 2 : 1 | 220 - 270 | 100 | 52,3 | 14,1 | 10,5 | 0,7 |
| 6 | 160 | 2 | 2 : 1 | 220 - 270 | 100 | 53,1 | 12,5 | 10,5 | 0,6 |
| 7 | 110 | 2 | 2 : 1 | 220 - 270 | 100 | 54,4 | 25,9 | 6,9 | 1,0 |
| 8 | 110 | 4 | 2 : 1 | 220 - 270 | 100 | 50,0 | 39,0 | 5,2 | 2,9 |
| 9 | 110 | 8 | 2 : 1 | 220 - 270 | 100 | 52,1 | 38,4 | 5,2 | 2,6 |
| 10 | 120 | 2 | 1 : 1 | 220 - 270 | 98,1 | 67,6 | 20,0 | 5,6 | 1,6 |
| 11 | 120 | 2 | 2 : 1 | 220 - 270 | 97,0 | 51,8 | 32,5 | 6,7 | 2,7 |
| 12 | 120 | 2 | 4 : 1 | 220 - 270 | 100 | 48,4 | 46,5 | 9,2 | 1,8 |
| 13 | 120 | 2 | 2 : 1 | 220 - 270 | 97 | 51,8 | 32,5 | 6,7 | 2,1 |
| 14 | 120 | 4 | 2 : 1 | 220 - 270 | 97,8 | 51,0 | 26,9 | 8,3 | 1,0 |
| 15 | 120 | 8 | 2 : 1 | 220 - 270 | 96,6 | 50,7 | 28,9 | 11,0 | 1,1 |
| 16 | 110 | 2 | 2 : 1 | 180 - 220 | 100 | 40,7 | 38,4 | 2,3 | 18,7 |
| 17 | 200 | 2 | Benzylalkohol 2 : 1 | 220 - 270 | 100 | 52,3 | 25,6 | 7,5 | 2,6 |
| 18 | 120 | 2 | 2 : 1 | Zeolith ZSM5 | 100 | 43,5 | 27,2 | 1,3 | 27,5 |
| 19 | 120 | 2 | 2 : 1 | < 110 | 100 | 50,3 | 27,2 | 1,3 | 26,2 |

4

**Patentansprüche**

1. Verfahren zur Herstellung von p-Aralkylbiphenylen durch Aralkylierung von Biphenyl, **dadurch gekennzeichnet**, daß die Aralkylierung an einem Katalysator vom Montmorillonit-Typ erfolgt, der eine spezifische Oberfläche von mindestens 110 m²/g hat.

2. Verfahren nach Anspruch 1, **dadurch gekennzeichnet,** daß der Montmorillonit eine spezifische Oberfläche von 110 bis 300 m²/g hat.

3. Verfahren nach Anspruch 2, **dadurch gekennzeichnet,** daß der Montmorillonit eine spezifische Oberfläche von 180 bis 280 m²/g hat.

4. Verfahren nach Anspruch 2, **dadurch gekennzeichnet,** daß der Montmorillonit eine spezifische Oberfläche von 220 bis 270 m²/g hat.

5. Verfahren nach einem der Ansprüche 1 bis 4, **dadurch gekennzeichnet,** daß die Aralkylierung mit Benzylalkohol oder Benzylchlorid erfolgt.


**Claims**

1. A process for preparing p-aralkylbiphenyls, by aralkylation of biphenyl, characterized in that the aralkylation takes place on a catalyst of the montmorillonite type, which has a specific surface of at least 110 m²/g.

2. A process according to Claim 1, characterized in that the montmorillonite has a specific surface of from 110 to 300 m²/g.

3. A process according to Claim 2, characterized in that the montmorillonite has a specific surface of from 180 to 280 m²/g.

4. A process according to Claim 2, characterized in that the montmorillonite has a specific surface of from 220 to 270 m²/g.

5. A process according to any one of Claims 1 to 4, characterized in that the aralkylation takes place with benzyl alcohol or benzyl chloride.


**Revendications**

1. Procédé pour la préparation de p-aralkylbiphényles par aralkylation du biphényle, caractérisé en ce que l'aralkylation s'effectue sur un catalyseur de montmorillonite qui présente une surface spécifique d'au moins 110 m²/g.

2. Procédé selon la revendication 1, caractérisé en ce que la montmorillonite présente une surface spécifique de 110 à 300 m²/g.

3. Procédé selon la revendication 2, caractérisé en ce que la montmorillonite présente une surface spécifique de 180 à 280 m²/g.

4. Procédé selon la revendication 2, caractérisé en ce que la montmorillonite présente une surface spécifique de 220 à 270 m²/g.

5. Procédé selon l'une des revendications 1 à 4, caractérisé en ce que l'aralkylation s'effectue avec de l'alcool de benzyle ou du chlorure de benzyle.